# EUROPEAN PATENT APPLICATION

(11) **EP 4 776 295 A1**
(43) Date of publication of application: **15.07.2026**
(21) Application number: 25203064.8
(22) Date of filing: 18.09.2025
(51) Int. Cl.: G16H 10/00, G16H 50/20, G16H 50/50

(54) **PREDICTING CARDIOLOGIST LEVEL CLINICAL EXPLANATIONS FOR ELECTROCARDIOGRAM SIGNAL CLASSIFICATION USING DEEP LEARNING**

(30) Priority: 13.01.2025 IN 202521002765
(71) Applicant: Tata Consultancy Services Limited, Maharashtra (IN)
(72) Inventor: JOSE, Akshay Kiran, 560066 Bangalore, Karnataka (IN); UKIL, Arijit, 700160 Kolkata, West Bengal (IN); DEB, Trisrota, 700160 Kolkata, West Bengal (IN); PAL, Arpan, 700160 Kolkata, West Bengal (IN)
(74) Representative: Goddar, Heinz J.

(57) **Abstract**

This disclosure relates generally to a method and system for predicting cardiologist level clinical explanations for electrocardiogram signal classification using deep learning. Conventional methods for explainability of artificial intelligence models are influenced by data distribution and do not provide clinical level explanations in cardiac healthcare. The method disclosed provides clinical level explanations for rhythm diagnosis from ECG signals. The method validates that clinical concepts in ECG signals can be extracted by generating a set of synthetic ECG signals. The set of synthetic ECG signals are generated using trained generative adversarial networks and statistical parameterization techniques. Further the set of synthetic ECG signals are validated by classifying and comparing them with a set of real ECG signals. Then a clinical concept identifier model is trained for predicting the clinical concepts present in an ECG signal classified into a set of signal classes. These clinical concepts predicted are cardiologist level explanations.

## Description

### CROSS-REFERENCE TO RELATED APPLICATIONS AND PRIORITY

The present application claims priority to Indian application no. 202521002765, filed on January 13, 2025.

### TECHNICAL FIELD

The disclosure herein generally relates to explainable artificial intelligence in cardiac healthcare, and, more particularly, to a method and system for predicting cardiologist level clinical explanations for electrocardiogram signal classification using deep learning.

### BACKGROUND

An Electrocardiogram (ECG) is a test that records the electrical activity of the heart and amounts to the most among all the ways used to detect and study the heart. The interpretation of ECG recordings is usually done in a clinical setting by a specialized medical professional to test for abnormal heart activity (arrhythmia) or presence of various cardiovascular diseases (CVDs). With rising risk of cardiac diseases, computerized decision systems have been sought to keep up with the rate at which cardiac monitoring had to progress, yet the subtle disposition of cardiac features prove challenging the early detection of disease-causing heart activity. Lately, Deep Learning (DL) driven decision making models have blazed a trail in many health-related sectors with its effectiveness and versatility. Yet, in high stakes situations, the adoption of such methods has been hindered by a variety of reasons.

The term eXplainable AI (XAI) in machine learning techniques enables human users to understand, trust, and effectively manage the emerging generation of artificially intelligent partners. XAI collates various lines of thought such as intelligibility, comprehensibility and explainability among others that must be incorporated into development of decision systems. Some decision systems such as rule based learners and linear models are interpretable by design whereas others like Multi-Layered Perceptrons (MLPs) are not. To explain the decisions of the latter, post-hoc explainability methods such as explanation by visualization, local explanations, explanations by simplification, explanation by examples are used. Model agnostic techniques such as Local Interpretable Model-Agnostic Explanations (LIME) and SHapley Additive exPlanations (SHAP) are widely used and have underwent several revisions. For deep learning techniques such as MLPs, there exist Deep Learning Important Features (DeepLIFT) that assigns contribution scores to neurons and for Convolutional Neural Networks (CNN), the suite of explanation techniques are more visual in nature owing to the model design that lends itself to such. In particular for CNNs, sensitivity analysis methods of the classifier output like saliency maps or insights derived from convolutional feature maps like Class Activation Maps (CAMs) are widely sought. CAMs indicate the discriminative regions of the input used by the CNN to identify a particular class, a notable variant of which is the Gradient-weighted Class Activation Mapping (Grad-CAM), which uses the gradients of any target class, flowing into the final convolutional layer to produce a coarse localization map, highlighting the important regions in the input for predicting the class. In cardiac healthcare domain, explanations are expected from XAI that cater to varied requirements, such as that of a cardiac specialist who validates the explanations and relays feedback, the developer who wants to tweak the model based on the feedback, a medical professional or a caregiver who makes uses of the explanations to tend to a patient and ultimately the patients themselves.

Generative AI (GenAI) as a virtual assistant is an enabler in providing explanations that are clinically relevant to the cardiovascular healthcare community. As a virtual assistant, it caters to the explainability needs of medical practitioners who are not cardiac specialists by means of evidence-based explanations, mimicking that of a cardiac specialist. Some GenAI applications particularly of interest in the medical domain currently are Large Language Models (LLMs) tailored for biomedical use cases which get trained on large publicly available biomedical literature, QA datasets and medical records. While medical LLMs can provide post-processing kind of clinical explanations, clinical explanations need to be developed which are integral part of the learning system.

### SUMMARY

Embodiments of the present disclosure present technological improvements as solutions to one or more of the above-mentioned technical problems recognized by the inventors in conventional systems. For example, in one embodiment, a method for predicting cardiologist level clinical explanations for electrocardiogram signal classification using deep learning is provided. The method includes generating a plurality of synthetic electrocardiogram (ECG) signals for validating whether a set of clinical concepts are embedded in a plurality of real ECG signals. This step for generating includes, initially segmenting a set of ECG signals received from a public dataset into a plurality of heartbeats. Further the plurality of heartbeats is grouped into a first set of bins. Each bin amongst the first set of bins comprises a set of heartbeats of equal vector length. Then a plurality of synthetic heartbeats is generated using a trained generative adversarial network (GAN) corresponding to the set of heartbeats in each bin amongst the first set of bins. Further the generated plurality of synthetic heartbeats is grouped into a second set of bins based on R-peak location of the plurality of synthetic heartbeats. The R-peak location of a set of synthetic heartbeats in each bin is in a pre-defined range of value. Then the plurality of synthetic ECG signals is generated from the generated plurality of synthetic heartbeats based on a statistical parameterization technique. Further the method includes, filtering the plurality of synthetic ECG signals to identify a plurality of realistic ECG signals based on a classification technique. Then the method includes, generating a plurality of indistinguishable ECG signals from the plurality of realistic ECG signals and a plurality of real ECG signals using the classification technique. Further the method includes validating the set of clinical concepts embedded in the plurality of indistinguishable ECG signals based on diagnosis of the plurality of indistinguishable ECG signals and the plurality of real ECG signals. Finally, the method includes training the clinical concept identifier model using a curated dataset comprising a plurality of labelled real ECG signals, for predicting the set of clinical concepts.

In another aspect, a system for predicting cardiologist level clinical explanations for electrocardiogram signal classification using deep learning is provided. The system comprises memory storing instructions; one or more communication interfaces; and one or more hardware processors coupled to the memory via the one or more communication interfaces, wherein the one or more hardware processors are configured by the instructions to generate a plurality of synthetic electrocardiogram (ECG) signals for validating whether a set of clinical concepts are embedded in a plurality of real ECG signals. This step for generating includes, initially segmenting a set of ECG signals received from a public dataset into a plurality of heartbeats. Further the plurality of heartbeats is grouped into a first set of bins. Each bin amongst the first set of bins comprises a set of heartbeats of equal vector length. Then a plurality of synthetic heartbeats is generated using a trained generative adversarial network (GAN) corresponding to the set of heartbeats in each bin amongst the first set of bins. Further the generated plurality of synthetic heartbeats is grouped into a second set of bins based on R-peak location of the plurality of synthetic heartbeats. The R-peak location of a set of synthetic heartbeats in each bin is in a pre-defined range of value. Then the plurality of synthetic ECG signals is generated from the generated plurality of synthetic heartbeats based on a statistical parameterization technique. Further the system includes, filtering the plurality of synthetic ECG signals to identify a plurality of realistic ECG signals based on a classification technique. Then the system includes, generating a plurality of indistinguishable ECG signals from the plurality of realistic ECG signals and a plurality of real ECG signals using the classification technique. Further the system includes validating the set of clinical concepts embedded in the plurality of indistinguishable ECG signals based on diagnosis of the plurality of indistinguishable ECG signals and the plurality of real ECG signals. Finally, the system includes training the clinical concept identifier model using a curated dataset comprising a plurality of labelled real ECG signals, for predicting the set of clinical concepts.

The system further predicts one or more clinical concepts from an ECG signal using the trained clinical concept identifier model. This step includes providing the ECG signal to the trained clinical concept identifier model and a trained rhythm classification model. Further this step includes parallelly performing classification of ECG signal by the trained rhythm classification model into a set of ECG signal classes and prediction of one or more clinical concepts by the trained clinical concept identifier model. The one or more clinical concepts are cardiologist level clinical explanations for the set of ECG signal classes. The set of clinical concepts comprises a) P wave missing, b) Irregular QRS complex rhythm, c) Presence of fibrillatory waves, d) Regular rhythm, and e) Presence of P waves.

A synthetic ECG signal amongst the plurality of synthetic ECG signals is generated from the generated plurality of synthetic heartbeats based on the statistical parameterization technique. The steps a to e for generating the synthetic ECG signal includes. Here step a includes, defining a statistical parameterization for the synthetic ECG signal to be generated. The statistical parameterization comprises an expected mean heartbeat, an expected standard deviation and number of heartbeats. Step b includes, creating a simulated list with a set of values satisfying the expected mean heartbeat and the expected standard deviation using a skewed normal distribution. Further step c includes, selecting a first heartbeat to append to an initialized list of heartbeats by randomly selecting a bin from the second set of bins and a synthetic heartbeat from the randomly selected bin. Step d includes, determining a next heartbeat from the plurality of synthetic heartbeats based on a peak-to-peak distance between the plurality of synthetic heartbeats using the defined statistical parameterization. Finally, step e includes joining the next heartbeat to the list of heartbeats. Step c and step d are iteratively repeated until the set of values in the simulated list is processed based on the peak-to-peak distance using the defined statistical parameterization. The plurality of synthetic ECG signals is generated from the plurality of synthetic heartbeats for each statistical parameterization of a set of defined statistical parameterizations using the statistical parameterization technique.

In yet another aspect, there is provided a computer program product comprising a non-transitory computer readable medium having a computer readable program embodied therein, wherein the computer readable program, when executed on a computing device causes the computing device for predicting cardiologist level clinical explanations by generating a plurality of synthetic electrocardiogram (ECG) signals for validating whether a set of clinical concepts are embedded in a plurality of real ECG signals. This step for generating includes, initially segmenting a set of ECG signals received from a public dataset into a plurality of heartbeats. Further the plurality of heartbeats is grouped into a first set of bins. Each bin amongst the first set of bins comprises a set of heartbeats of equal vector length. Then a plurality of synthetic heartbeats is generated using a trained generative adversarial network (GAN) corresponding to the set of heartbeats in each bin amongst the first set of bins. Further the generated plurality of synthetic heartbeats is grouped into a second set of bins based on R-peak location of the plurality of synthetic heartbeats. The R-peak location of a set of synthetic heartbeats in each bin is in a pre-defined range of value. Then the plurality of synthetic ECG signals is generated from the generated plurality of synthetic heartbeats based on a statistical parameterization technique. Further the computer readable program includes, filtering the plurality of synthetic ECG signals to identify a plurality of realistic ECG signals based on a classification technique. Then the computer readable program includes, generating a plurality of indistinguishable ECG signals from the plurality of realistic ECG signals and a plurality of real ECG signals using the classification technique. Further the computer readable program includes validating the set of clinical concepts embedded in the plurality of indistinguishable ECG signals based on diagnosis of the plurality of indistinguishable ECG signals and the plurality of real ECG signals. Finally, the computer readable program includes training the clinical concept identifier model using a curated dataset comprising a plurality of labelled real ECG signals, for predicting the set of clinical concepts.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary block diagram of a system for predicting cardiologist level clinical explanations for electrocardiogram signal classification using deep learning according to some embodiments of the present disclosure.
FIG. 2A and FIG.2B, collectively referred as FIG.2, is an exemplary flow diagram illustrating a method for predicting cardiologist level clinical explanations for electrocardiogram signal classification using deep learning according to some embodiments of the present disclosure.
FIG. 3 is an exemplary flow diagram for generating a plurality of synthetic heartbeats using trained generative adversarial network (GAN) according to some embodiments of the present disclosure.
FIG. 4 is an exemplary flow diagram for generating a plurality of synthetic ECG signals from the generated plurality of synthetic heartbeats based on a statistical parameterization technique according to some embodiments of the present disclosure.
FIG. 5 is an overall block diagram for predicting cardiologist level clinical explanations for electrocardiogram signal classification using deep learning according to some embodiments of the present disclosure.
FIG.6A and FIG.6B is an example illustration of indistinguishable atrial fibrillation (AF) signal and real AF signal according to some embodiments of the present disclosure.
FIG.7A and FIG.7B are example illustrations of indistinguishable Normal Sinus Rhythm (NSR) signal and real NSR signal according to some embodiments of the present disclosure.
FIG. 8 are example illustrations of three ECG signals predicted by a clinical concept identifier model comprising irregular QRS complex rhythm, no definite P wave and presence of fibrillatory waves according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Embodiments of the present disclosure provide a method for clinically valid concept-based explanations generated by deep learning model referred to as a clinical concept identifier model and diagnosis made by a rhythm classifier model. The method disclosed provides cardiology domain-specific explanation that transcends morphological boundaries and provides explanations that maintain consistency with a clinical diagnosis setting. The method disclosed, using a rhythm classification model classifies an electrocardiogram signal (ECG) into a set of classes like atrial fibrillation (AF) signal and normal sinus rhythm (NSR) signal. Further the method using the clinical concept identifier model that predicts a set of clinical concepts, which are cardiologist level clinical explanations, present in each class of the ECG signal. However, to validate whether the set of clinical concepts are present in the ECG signal, the method disclosed initially performs a validation such that it is proved that the set of clinical concepts can be extracted from the ECG signal using a model. This validation is performed by generating a set of synthetic ECG signals embedded with the set of clinical concepts. The set of synthetic ECG signals are generated using trained generative adversarial networks and statistical parameterization techniques. Then initially using a machine learning model and further with the help of a cardiologist these generated set of synthetic ECG signals are validated with that of real ECG signals. Thus, it is proved that the set of clinical concepts can be extracted from the real ECG signals which is provided as clinical level explanations for ECG signal classification.

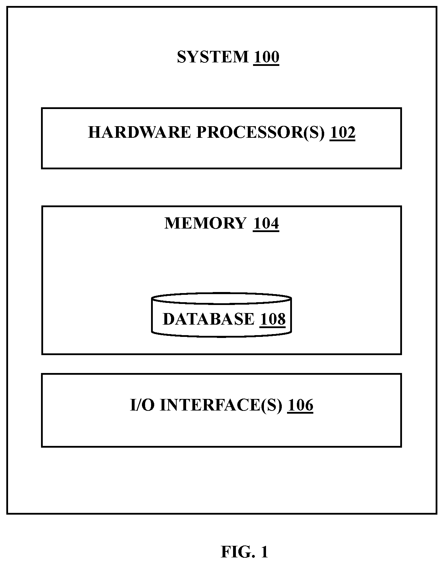

Referring now to the drawings, and more particularly to FIG. 1 through FIG. 8, where similar reference characters denote corresponding features consistently throughout the figures, there are shown preferred embodiments and these embodiments are described in the context of the following exemplary system and/or method.

FIG. 1 illustrates an exemplary block diagram of a system for predicting cardiologist level clinical explanations for electrocardiogram signal classification using deep learning according to some embodiments of the present disclosure. In an embodiment, the system 100 includes one or more hardware processors 102, communication interface(s) or input/output (I/O) interface(s) 106, and one or more data storage devices or memory 104 operatively coupled to the one or more processors 102. The one or more hardware processors 102 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of computing systems, such as laptop computers, notebooks, hand-held devices, workstations, mainframe computers, servers, a network cloud and the like.

The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) can include one or more ports for connecting a number of devices to one another or to another server.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

In an embodiment, the memory 104 includes a plurality of modules, such as synthetic ECG signal generator module, clinical concept identifier module, rhythm classification module (not shown), and the like. Further, the plurality of modules includes programs or coded instructions that supplement applications or functions performed by the system 100 for executing different steps involved in the process for measuring social awareness by the system 100. The plurality of modules, amongst other things, can include routines, programs, objects, components, and data structures, which performs particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by the one or more hardware processors 102, or by a combination thereof. The plurality of modules can include various sub-modules (not shown).

Further, the memory 104 may include a database 108 or repository. The memory 104 may comprise information pertaining to input(s)/output(s) of each step performed by the processor(s) 102 of the system 100 and methods of the present disclosure. In an embodiment, the database 108 may be external (not shown) to the system 100 and coupled via the I/O interface 106. The database may include data sources corresponding public dataset, curated dataset etc.

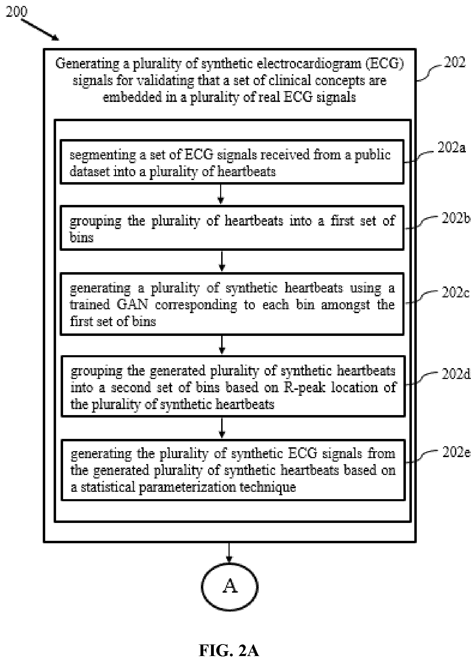

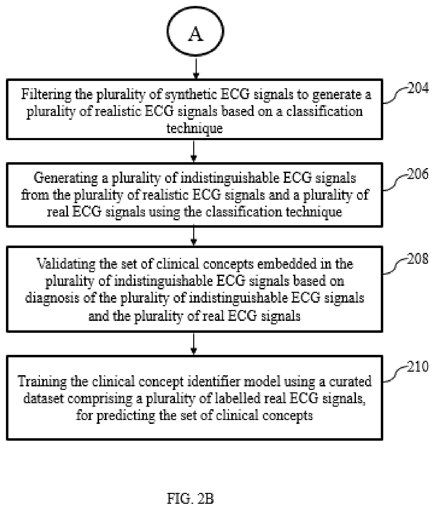

FIG. 2A and FIG.2B, collectively referred as FIG.2, is an exemplary flow diagram illustrating a method for predicting cardiologist level clinical explanations for electrocardiogram signal classification using deep learning according to some embodiments of the present disclosure. In an embodiment, the system 100 comprises one or more data storage devices or the memory 104 operatively coupled to the one or more hardware processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1, and the steps of flow diagram as depicted in FIG. 2. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIG. 2A, at step 202 of the method 200, the one or more hardware processors 102 are configured to generate a plurality of synthetic electrocardiogram (ECG) signals using the synthetic ECG signal generator module for validating whether a set of clinical concepts are embedded in a plurality of real ECG signals. The step 202a through 202e explains generation of the plurality of synthetic ECG signals. At step 202a, a set of ECG signals are segmented to a plurality of heartbeats. The set of ECG signals are received from a public dataset. The set of ECG signals are segmented into various heartbeat chunks. For example, if an ECG signal comprises 5 heartbeats, it is segmented into 5 different heartbeat chunks. Each heartbeat chunk is called as a heartbeat. Thus, thousands of heartbeats are segmented from the set of ECG signals. Here a fixed window for only the first heartbeat is taken from then it takes the chunk from where the previous beat ends.

At step 202b, the plurality of heartbeats is grouped into a first set of bins. The plurality of heartbeats is grouped into different bins based on their lengths or durations. Some heartbeats may be less than a second long, some will be half a second long and so on. After this step, considering the example above, 5 bins are created, where each bin has heartbeats of similar length. For instance, bin 1 contains beats less than 0.5 seconds long. Instead of mentioning as "seconds", it can alternatively be called as every beat as a sequence of numbers (called as a vector). Here as an instance, a 1-second-long beat is represented by 300 length vector. So, a 0.5 sec long beat is represented by 150 length vector.

Further at step 202c, a plurality of synthetic heartbeats is generated using a trained generative adversarial network (GAN) corresponding to each bin amongst the first set of bins. Here one GAN is trained for each bin amongst the first set of bins, to generate the synthetic heartbeats. Here the heartbeats in a specific bin are all padded or trimmed to a common length before providing to the corresponding trained GAN. The corresponding GAN generates synthetic heartbeats as output with the common length. Thus, the plurality of synthetic heartbeats is generated from a set of trained GANs. The GAN is trained to learn to generate synthetic heartbeats. For example, suppose bin 1 has a total of 100 beats, then the trained GAN learns to generate heartbeats that look similar and generate countless such heartbeats.

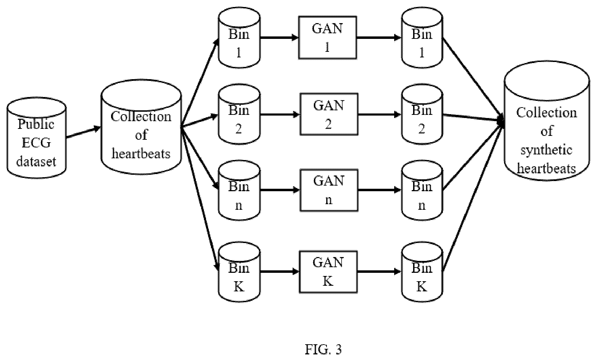

Next at step 202d, the generated plurality of synthetic heartbeats is grouped into a second set of bins based on R-peak location of the plurality of synthetic heartbeats. This grouping is done with respect to R-peak location. For example, in a 1-second-long beat, (i.e., 300 length-vector,) the R-peak might appear half-way through, (i.e., at 150th location of the vector). This is made as one group. Again, as an example, all heartbeats (irrespective of length) that have the R-peak location at 100th or less will be put in another group. Again, if the R-peak likes between 100 and 120, those heartbeats will be kept in the next bin, and so on. At the end of this step, there will be K number of bins. 1^{st} bin will have those heartbeats with R-peak location lesser than 100, 2^{nd} bin will have beats with R-peak location greater than 100 and less than 120 and so on. The above description as depicted in FIG. 3 is an exemplary flow diagram for generating a plurality of synthetic heartbeats using trained generative adversarial network (GAN) in accordance with some embodiments of the present disclosure.

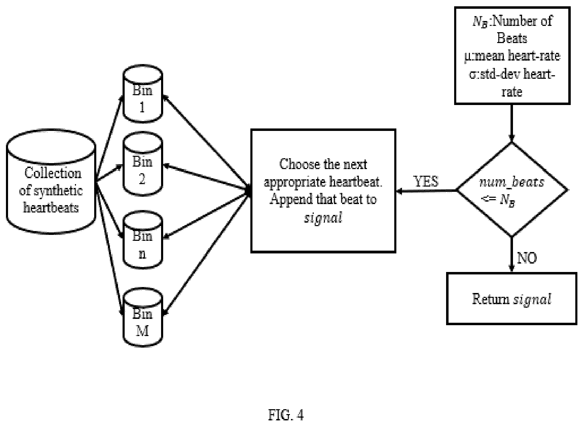

Finally at step 202e, the plurality of synthetic ECG signals is generated from the generated plurality of synthetic heartbeats based on a statistical parameterization technique. Before joining the synthetic heartbeats end-on-end, a set of statistical parameters need to be defined, and the synthetic ECG signal need to be generated in agreeable with the set of statistical parameters. To generate the synthetic ECG signal from the plurality of synthetic heartbeats, a statistical parameterization comprising the set of statistical parameters is defined. The set of statistical parameters comprises an expected mean heartbeat, an expected standard deviation and number of heart-beats. Once the statistical parameters are agreed, a possible sequence of heartbeats are simulated that satisfy the defined statistical parameterization. This is called sampling from a particular distribution that satisfies the statistical parameters and is called as Simbeats. For example, if an ECG signal with 6 heartbeats with a mean of 82 and standard deviation of 5 bpm is needed, then a possible value for Simbeats is [77,80,82,84,87]. This simulated list is a set of values drawn from a skewed normal distribution defined by the expected mean and the expected standard deviation defined. This means that the 1^{st} and 2^{nd} heartbeats together should have a heart rate of 77, 2^{nd} and 3^{rd} heartbeats together should appear as having a heart rate of 80 and so on. To do the above, a first heartbeat is selected and appended to an initialized list of heartbeats by randomly selecting a bin from the second set of bins and randomly selecting a heartbeat from the randomly selected bin. Then a next heartbeat to be appended is determined from the plurality of synthetic heartbeats based on a peak-to-peak distance between the plurality of synthetic heartbeats using the defined statistical parameterization. Further the next heartbeat is appended with the list of heartbeats. These steps of determining next heartbeat and joining with the list of heartbeats is iteratively repeated until all the values in the simulated list are processed. Finally, after this, the plurality of synthetic ECG signals is generated from the plurality of synthetic heartbeats for each statistical parameterization of a set of defined statistical parameterizations using the statistical parameterization technique. Each synthetic ECG signal will have a specific defined statistical parameterization being satisfied. In accordance with the explanation as above, FIG. 4 shows an exemplary flow diagram for generating a plurality of synthetic ECG signals from the generated plurality of synthetic heartbeats based on a statistical parameterization technique in accordance with some embodiments of the present disclosure. The algorithm for generating the plurality of synthetic ECG signals is provided as below, where *S̃* is the plurality of synthetic heartbeats,

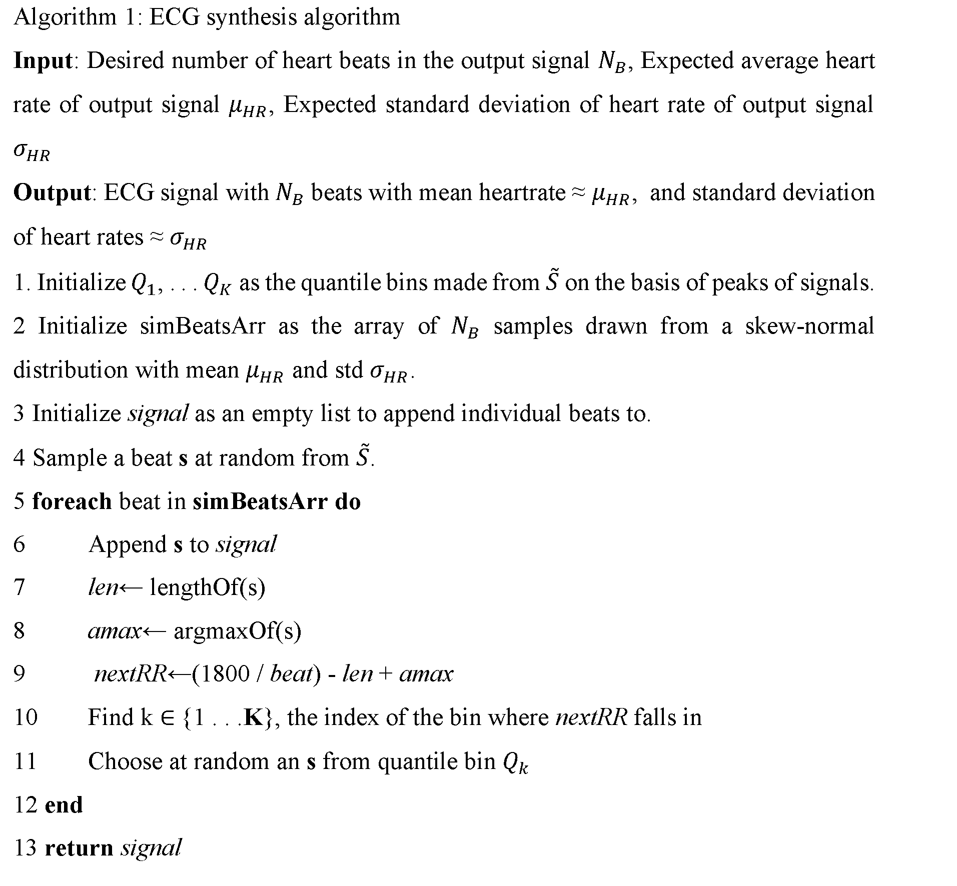

Next at step 204 of the method 200, the one or more hardware processors 102 are configured to filter the plurality of synthetic ECG signals to generate a plurality of realistic ECG signals based on a classification technique. With the synthetic ECG signals generated, it is not immediately clear if some synthetic ECG signals are not just random collection of heartbeats, or it encodes useful clinical concepts within it. Due to the inherent random nature of the steps so far explained above, (i.e., creating heartbeats using trained GANs and selecting heartbeats to add end-on-end) it might happen that some synthetic ECG signals among the generated synthetic ECG signals may not be very compliant to ECG standards. So, it becomes essential to find out which synthetic ECG signals in the generated plurality of synthetic ECG signals embed concept information in them, and which do not. The generated plurality of synthetic ECG signals that qualify for the test must meet some criterion to be considered realistic and indistinguishable-to-real. So, first the plurality of synthetic ECG signals is filtered out to generate the plurality of realistic ECG signals using a classification technique. Here, for filtering, first the plurality of synthetic ECG signals is passed through a trained classifier model to check if the plurality of synthetic ECG signals is classified to a set of classes, such as atrial fibrillation (AF) signal or normal sinus rhythm (NSR) signals with high confidence. It is to be noted that the classification to the set of classes is not limited to AF signal or NSR signal classification. However, henceforth these two classes such as AF and NSR is being considered for explaining the process. As an example, suppose the generated synthetic ECG signal **x1** goes through the classifier model and it predicts 90% chance that the signal is AF. This proves that the generated synthetic ECG signal **x1** is not just a random collection of heartbeats. As such each synthetic ECG signal is fed into this process and those signals that were classified as either AF or NSR with high probability are selected for further processing. These filtered out signals are the plurality of realistic ECG signals.

Further at step 206 of the method 200, the one or more hardware processors 102 are configured to generate a plurality of indistinguishable ECG signals from the plurality of realistic ECG signals and a plurality of real ECG signals using the classification technique. Here the method disclosed evaluates how close-to-real some of the plurality of realistic ECG signals are. For this, the scores returned by the same classifier model on each realistic signal in the plurality of realistic ECG signals are compared with the scores for some real AF or NSR signals. If a realistic ECG signal has a score that is very close to some real signals, it is concluded that the realistic ECG signal embeds similar clinical concepts to that of those real signals. These realistic ECG signals which have a closer value after comparison is considered and they are identified as the plurality of indistinguishable ECG signals.

Next at step 208 of the method 200, the one or more hardware processors 102 are configured to validate the set of clinical concepts embedded in the plurality of indistinguishable ECG signals based on diagnosis of the plurality of indistinguishable ECG signals and the plurality of real ECG signals. Here, the plurality of indistinguishable signals is presented before a cardiologist under the scenario that they need to be diagnosed for rhythm (AF or NSR). For this, 10 generated AF signals and 10 generated NSR signals are presented along with 10 each of real AF and NSR signals (a total of 40 signals) before a cardiologist. The result was that the cardiologist was able to rightly determine the rhythm associated with each signal and did not doubt the authenticity of any signal. It is important for ECG synthesis such that if it is proved that the clinical concepts can be embedded into an ECG signal, thereby making it a pseudo-realistic instance of a particular rhythm, it describes the ECG signal as a collection of concepts. If the clinical concepts of Atrial Fibrillation (AF) can be embedded into a generated signal and if the signal gets accepted as one, the understanding of the signal by a cardiologist was the same as it would have been with a real AF signal. This shows that the real AF signals and generated AF signals were not seen as different by the cardiologist. Being able to embed such clinical concepts artificially suggests that it can extract the concept information directly from the ECG signal.

After validating that the set of clinical concepts is embedded and can be extracted from the ECG signal, at step 210 of the method 200, the one or more hardware processors 102 are configured to train the clinical concept identifier model using a curated dataset comprising a plurality of labelled real ECG signals using the clinical concept identifier module. The trained clinical concept identifier model is used for predicting the set of clinical concepts. A deep learning model M_{E} (the clinical concept identifier model) is trained to predict the set of clinical concepts present in an ECG signal using the curated dataset. The curated dataset comprises the plurality of labelled real ECG signals whose rhythm was either AF or NSR. The cardiologist diagnosed the rhythm and gave short reasons for their diagnosis which were explanations given in ECG terminology. They were a) P wave missing, b) Irregular QRS complex rhythm, c) Presence of fibrillatory waves, for a diagnosis of AF and d) Regular rhythm, and e) Presence of P waves for a diagnosis of NSR. The M_{E} model takes a signal in and predicts which (one or more) among 'P wave missing', 'Irregular QRS complex rhythm', 'presence of fibrillatory waves', 'Regular rhythm', 'presence of P waves'.

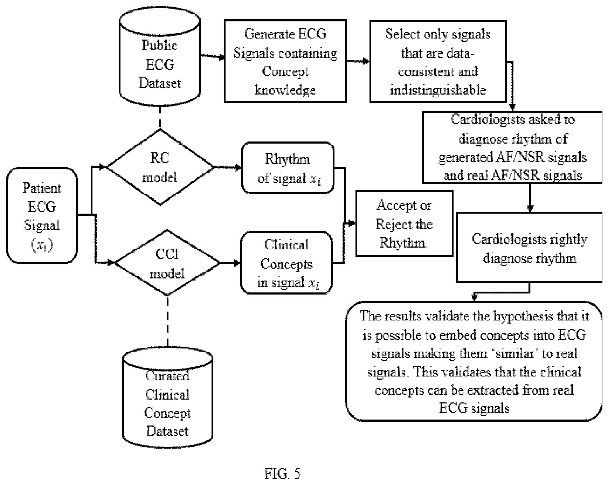

Further for testing the clinical concept identifier model, one or more clinical concepts are predicted from an ECG signal which is provided to the trained clinical concept identifier model. Here the ECG signal is provided to the trained clinical concept identifier model and a trained rhythm classification model M_{R}. Parallelly, the ECG signal is classified by the trained rhythm classification model using the rhythm classification module into a set of ECG signal classes like AF signal or NSR signal. The rhythm classification model needs ECG signals that need only be labelled for their rhythm type, namely AF or NSR. These labelled ECG signals are used for training the rhythm classifier model, which is a typical classification model. Also, the one or more clinical concepts are predicted by the trained clinical concept identifier model. These one or more clinical concepts are cardiologist level clinical explanations for the set of ECG signal classes. The overall steps explained till now are shown as a block diagram in FIG. 5. FIG. 5 is an overall block diagram for predicting cardiologist level clinical explanations for electrocardiogram signal classification using deep learning in accordance with some embodiments of the present disclosure.

EXPERMENTAL RESULTS: Let G denote the various synthetic signals and to test them under a machine learning perspective on realism and indistinguishability, first the rhythm predictions of each signal in G under *M_{R}*, are found and only selected for the next step, signals that exhibit either NSR or AF predictions for all prediction steps with arbitrarily high confidence. *M_{R}*, is a classifier model with a 34-layer CNN architecture. It takes as input a time-series of raw ECG signal, and outputs a sequence of label predictions, almost once per second's worth of input time-series. *M_{R}*, takes in a time-series signal and outputs a classification probability score for each 1second time-series frame/segment. For a 30 second signal, 30 different scores are received as output. For a generated signal, if every such segment has high scores for AF, that makes it a realistic AF signal. Similarly, for NSR signal. To compare between signal s1 and s2, the scores of the corresponding segment are compared for closeness. If each segment shows similar scores between two signals, they are termed as indistinguishable from each other.

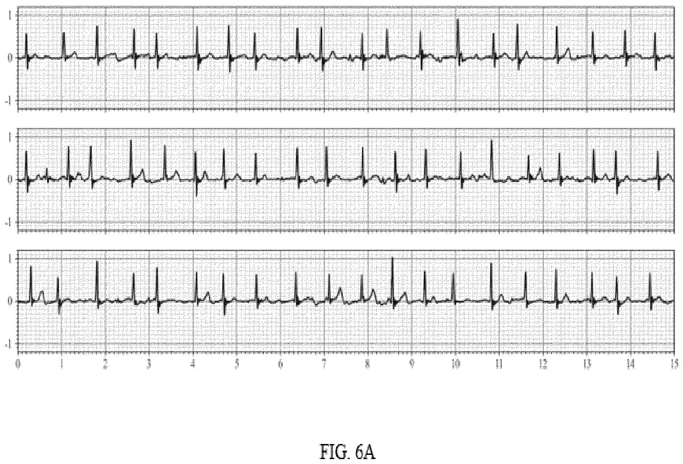

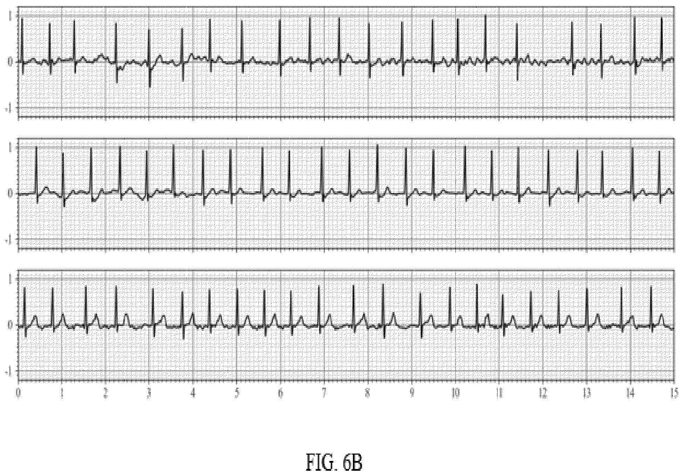

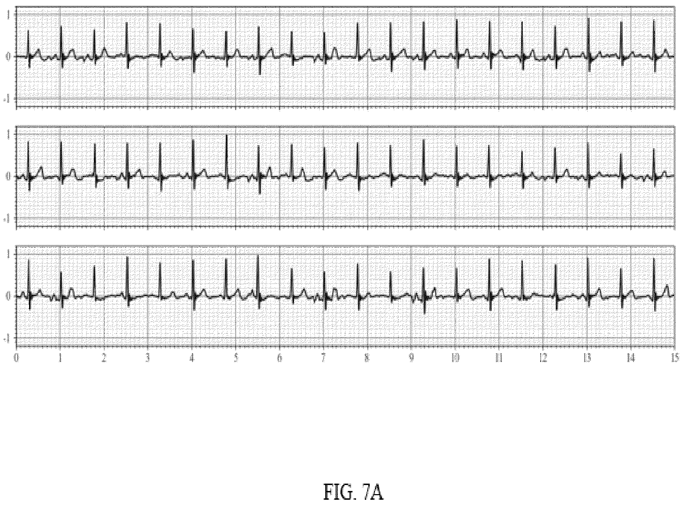

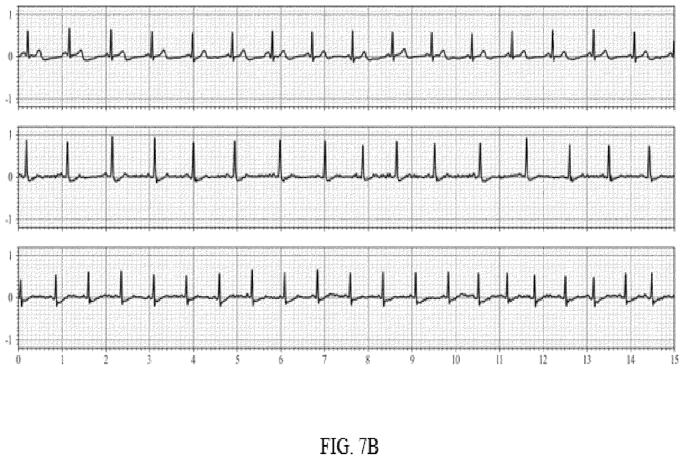

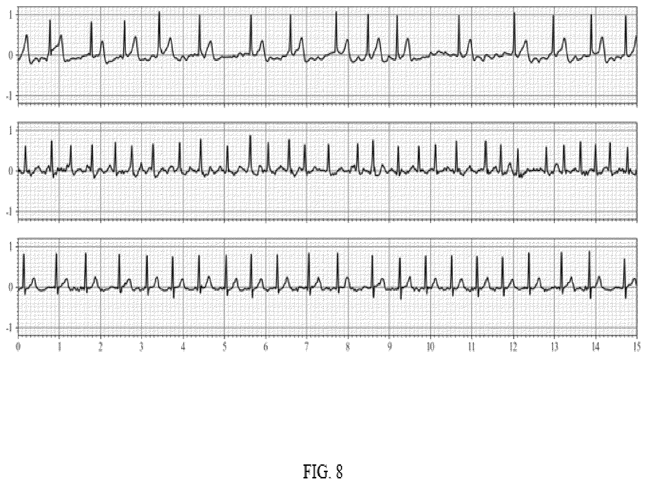

The set of signals that make through form *G_{realistic}* ⊂ G. At the next step, the sequence of predictions of each signal in *G_{realistic}* is compared against that of real signals under *M_{R}*, . It is defined as *G_{indist}* ⊂ *G_{realistic},* the set of signals from *G_{realistic}* that show arbitrary closeness under ∥ *L* ∥₁ of predictions as the set of indistinguishable generated signals. 20 synthetic signals from *G_{indist}* are presented along with 20 real signals from Physionet2017, both equal proportions of AF and NSR to cardiologists under the guise of rhythm diagnosis. The outcome of the blind-experiment was that they were able to distinguish AF signals from NSR signals without the distinction of which was real and which was synthetic. No discussion came up regarding the origin of any signal and that bodes well with the attempt at generating realistic ECG signals that maintain information about the clinical concepts they represent. Having passed through two phases of testing for realism and indistinguishability, one at the ML level under the model and at the clinical level under the diagnosis of a cardiologist, the blind experiment corroborates the desired use of generative AI as a concept mapper or concept builder. FIG. 6A through FIG.7B shows the indistinguishable AF, NSR signals and the real AF, NSR signals. FIG.6A and FIG.6B is an example illustration of indistinguishable atrial fibrillation (AF) signal and real AF signal according to some embodiments of the present disclosure. FIG.7A and FIG.7B are example illustrations of indistinguishable Normal Sinus Rhythm (NSR) signal and real NSR signal according to some embodiments of the present disclosure. FIG. 8 are example illustrations of three ECG signals predicted by a clinical concept identifier model comprising irregular QRS complex rhythm, no definite P wave and presence of fibrillatory waves, the clinical concepts of AF signal according to some embodiments of the present disclosure. These clinical concepts are predicted by the clinical concept identifier model that are relevant in clinical aspect.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

Embodiments of the present disclosure provides a method for predicting cardiologist level clinical explanations for ECG signal classification. The method disclosed initially validates that the set of clinical concepts can be extracted from a real ECG signal by generating synthetic ECG signals. The synthetic ECG signals are generated using synthetic heartbeats generated utilizing trained GANs and statistical parameterization techniques. Further validating, that the set of clinical concepts can be extracted from the synthetic ECG signals, a clinical concept identifier model is trained with the set of clinical concepts for clinical concept-based explanations. Also, diagnosis of the real ECG signals is performed using a rhythm classifier model.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200) comprising:
generating (202), via one or more hardware processors, a plurality of synthetic electrocardiogram (ECG) signals for validating whether a set of clinical concepts are embedded in a plurality of real ECG signals, wherein the step comprises:
segmenting a set of ECG signals received from a public dataset into a plurality of heartbeats (202a);
grouping the plurality of heartbeats into a first set of bins, wherein each bin amongst the first set of bins comprises a set of heartbeats of equal vector length (202b);
generating a plurality of synthetic heartbeats using a trained generative adversarial network (GAN) corresponding to the set of heartbeats in each bin amongst the first set of bins (202c);
grouping the generated plurality of synthetic heartbeats into a second set of bins based on R-peak location of the plurality of synthetic heartbeats, wherein the R-peak location of a set of synthetic heartbeats in each bin is in a pre-defined range of value (202d); and
generating the plurality of synthetic ECG signals from the generated plurality of synthetic heartbeats based on a statistical parameterization technique (202e);
filtering (204), via the one or more hardware processors, the plurality of synthetic ECG signals to identify a plurality of realistic ECG signals based on a classification technique;
generating (206), via the one or more hardware processors, a plurality of indistinguishable ECG signals from the plurality of realistic ECG signals and a plurality of real ECG signals using the classification technique;
validating (208), via the one or more hardware processors, the set of clinical concepts embedded in the plurality of indistinguishable ECG signals based on diagnosis of the plurality of indistinguishable ECG signals and the plurality of real ECG signals; and
training (210), via the one or more hardware processors, the clinical concept identifier model using a curated dataset comprising a plurality of labelled real ECG signals, for predicting the set of clinical concepts.

2. The processor implemented method as claimed in claim 1, comprising predicting one or more clinical concepts from an ECG signal using the trained clinical concept identifier model, wherein predicting the one or more clinical concepts comprises:
providing, via the one or more hardware processors, the ECG signal to the trained clinical concept identifier model and a trained rhythm classification model; and
parallelly performing, via the one or more hardware processors,
classifying the ECG signal by the trained rhythm classification model into a set of ECG signal classes; and
predicting the one or more clinical concepts by the trained clinical concept identifier model, wherein the one or more clinical concepts are cardiologist level clinical explanations for the set of ECG signal classes.

3. The processor implemented method as claimed in claim 1, wherein the set of clinical concepts comprises a) P wave missing, b) Irregular QRS complex rhythm, c) Presence of fibrillatory waves, d) Regular rhythm, and e) Presence of P waves.

4. The processor implemented method as claimed in claim 1, wherein generating a synthetic ECG signal amongst the plurality of synthetic ECG signals from the generated plurality of synthetic heartbeats based on the statistical parameterization technique comprises:
a) defining, via the one or more hardware processors, a statistical parameterization for the synthetic ECG signal to be generated, wherein the statistical parameterization comprising an expected mean heartbeat, an expected standard deviation and number of heartbeats;
b) creating, via the one or more hardware processors, a simulated list with a set of values satisfying the expected mean heartbeat and the expected standard deviation using a skewed normal distribution;
c) selecting a first heartbeat to append to an initialized list of heartbeats by randomly selecting, via the one or more hardware processors,
a bin from the second set of bins; and
a synthetic heartbeat from the randomly selected bin;
d) determining, via the one or more hardware processors, a next heartbeat from the plurality of synthetic heartbeats based on a peak-to-peak distance between the plurality of synthetic heartbeats using the defined statistical parameterization;
e) joining, via the one or more hardware processors, the next heartbeat to the list of heartbeats, wherein the step c and step d are iteratively repeated until the set of values in the simulated list is processed based on the peak-to-peak distance using the defined statistical parameterization.

5. The processor implemented method as claimed in claim 1, wherein the plurality of synthetic ECG signals is generated from the plurality of synthetic heartbeats for each statistical parameterization of a set of defined statistical parameterizations using the statistical parameterization technique.

6. A system (100), comprising:
a memory (104) storing instructions;
one or more communication interfaces (106); and
one or more hardware processors (102) coupled to the memory (104) via the one or more communication interfaces (106), wherein the one or more hardware processors (102) are configured by the instructions to:
generate a plurality of synthetic electrocardiogram (ECG) signals for validating whether a set of clinical concepts are embedded in a plurality of real ECG signals, wherein the step comprises:
segmenting a set of ECG signals received from a public dataset into a plurality of heartbeats;
grouping the plurality of heartbeats into a first set of bins, wherein each bin amongst the first set of bins comprises a set of heartbeats of equal vector length;
generating a plurality of synthetic heartbeats using a trained generative adversarial network (GAN) corresponding to the set of heartbeats in each bin amongst the first set of bins;
grouping the generated plurality of synthetic heartbeats into a second set of bins based on R-peak location of the plurality of synthetic heartbeats, wherein the R-peak location of a set of synthetic heartbeats in each bin is in a pre-defined range of value; and
generating the plurality of synthetic ECG signals from the generated plurality of synthetic heartbeats based on a statistical parameterization technique;
filter the plurality of synthetic ECG signals to identify a plurality of realistic ECG signals based on a classification technique;
generate a plurality of indistinguishable ECG signals from the plurality of realistic ECG signals and a plurality of real ECG signals using the classification technique;
validate the set of clinical concepts embedded in the plurality of indistinguishable ECG signals based on diagnosis of the plurality of indistinguishable ECG signals and the plurality of real ECG signals; and
train the clinical concept identifier model using a curated dataset comprising a plurality of labelled real ECG signals, for predicting the set of clinical concepts.

7. The system as claimed in claim 1, wherein one or more clinical concepts are predicted from an ECG signal using the trained clinical concept identifier model by:
providing the ECG signal to the trained clinical concept identifier model and a trained rhythm classification model; and
parallelly performing
classifying the ECG signal by the trained rhythm classification model into a set of ECG signal classes; and
predicting the one or more clinical concepts by the trained clinical concept identifier model, wherein the one or more clinical concepts are cardiologist level clinical explanations for the set of ECG signal classes.

8. The system as claimed in claim 6, wherein the set of clinical concepts comprises a) P wave missing, b) Irregular QRS complex rhythm, c) Presence of fibrillatory waves, d) Regular rhythm, and e) Presence of P waves.

9. The system as claimed in claim 6, wherein generating a synthetic ECG signal amongst the plurality of synthetic ECG signals from the generated plurality of synthetic heartbeats based on the statistical parameterization technique comprises:
a) defining a statistical parameterization for the synthetic ECG signal to be generated, wherein the statistical parameterization comprising an expected mean heartbeat, an expected standard deviation and number of heartbeats;
b) creating a simulated list with a set of values satisfying the expected mean heartbeat and the expected standard deviation using a skewed normal distribution;
c) selecting a first heartbeat to append to an initialized list of heartbeats by randomly selecting,
a bin from the second set of bins; and
a synthetic heartbeat from the randomly selected bin;
d) determining a next heartbeat from the plurality of synthetic heartbeats based on a peak-to-peak distance between the plurality of synthetic heartbeats using the defined statistical parameterization; and
e) joining the next heartbeat to the list of heartbeats, wherein the step c and step d are iteratively repeated until the set of values in the simulated list is processed based on the peak-to-peak distance using the defined statistical parameterization.

10. The system as claimed in claim 6, wherein the plurality of synthetic ECG signals is generated from the plurality of synthetic heartbeats for each statistical parameterization of a set of defined statistical parameterizations using the statistical parameterization technique.

11. One or more non-transitory machine-readable information storage mediums comprising one or more instructions which when executed by one or more hardware processors cause:
generating a plurality of synthetic electrocardiogram (ECG) signals for validating whether a set of clinical concepts are embedded in a plurality of real ECG signals, wherein the step comprises:
segmenting a set of ECG signals received from a public dataset into a plurality of heartbeats;
grouping the plurality of heartbeats into a first set of bins, wherein each bin amongst the first set of bins comprises a set of heartbeats of equal vector length;
generating a plurality of synthetic heartbeats using a trained generative adversarial network (GAN) corresponding to the set of heartbeats in each bin amongst the first set of bins;
grouping the generated plurality of synthetic heartbeats into a second set of bins based on R-peak location of the plurality of synthetic heartbeats, wherein the R-peak location of a set of synthetic heartbeats in each bin is in a pre-defined range of value; and
generating the plurality of synthetic ECG signals from the generated plurality of synthetic heartbeats based on a statistical parameterization technique;
filtering the plurality of synthetic ECG signals to identify a plurality of realistic ECG signals based on a classification technique;
generating a plurality of indistinguishable ECG signals from the plurality of realistic ECG signals and a plurality of real ECG signals using the classification technique;
validating the set of clinical concepts embedded in the plurality of indistinguishable ECG signals based on diagnosis of the plurality of indistinguishable ECG signals and the plurality of real ECG signals; and
training the clinical concept identifier model using a curated dataset comprising a plurality of labelled real ECG signals, for predicting the set of clinical concepts.

12. The one or more non-transitory machine-readable information storage mediums as claimed in claim 11, wherein the one or more instructions which when executed by the one or more hardware processors cause predicting one or more clinical concepts from an ECG signal using the trained clinical concept identifier model, wherein predicting the one or more clinical concepts comprises:
providing the ECG signal to the trained clinical concept identifier model and a trained rhythm classification model; and
parallelly performing,
classifying the ECG signal by the trained rhythm classification model into a set of ECG signal classes; and
predicting the one or more clinical concepts by the trained clinical concept identifier model, wherein the one or more clinical concepts are cardiologist level clinical explanations for the set of ECG signal classes.

13. The one or more non-transitory machine-readable information storage mediums as claimed in claim 11, wherein the set of clinical concepts comprises a) P wave missing, b) Irregular QRS complex rhythm, c) Presence of fibrillatory waves, d) Regular rhythm, and e) Presence of P waves.

14. The one or more non-transitory machine-readable information storage mediums as claimed in claim 11, wherein generating a synthetic ECG signal amongst the plurality of synthetic ECG signals from the generated plurality of synthetic heartbeats based on the statistical parameterization technique comprises:
a) Defining a statistical parameterization for the synthetic ECG signal to be generated, wherein the statistical parameterization comprising an expected mean heartbeat, an expected standard deviation and number of heartbeats;
b) creating a simulated list with a set of values satisfying the expected mean heartbeat and the expected standard deviation using a skewed normal distribution;
c) selecting a first heartbeat to append to an initialized list of heartbeats by randomly selecting, via the one or more hardware processors,
a bin from the second set of bins; and
a synthetic heartbeat from the randomly selected bin;
d) determining a next heartbeat from the plurality of synthetic heartbeats based on a peak-to-peak distance between the plurality of synthetic heartbeats using the defined statistical parameterization;
e) joining the next heartbeat to the list of heartbeats, wherein the step c and step d are iteratively repeated until the set of values in the simulated list is processed based on the peak-to-peak distance using the defined statistical parameterization.

15. The one or more non-transitory machine-readable information storage mediums as claimed in claim 11, wherein the plurality of synthetic ECG signals is generated from the plurality of synthetic heartbeats for each statistical parameterization of a set of defined statistical parameterizations using the statistical parameterization technique.
